(19) Europäisches Patentamt
European Patent Office
Office européen des brevets

(11) EP 0 649 405 B1

(12) **EUROPÄISCHE PATENTSCHRIFT**

(45) Veröffentlichungstag und Bekanntmachung des Hinweises auf die Patenterteilung:
**26.03.1997 Patentblatt 1997/13**

(21) Anmeldenummer: **93915800.2**

(22) Anmeldetag: **30.06.1993**

(51) Int Cl.[6]: **C07C 401/00**, A61K 31/59

(86) Internationale Anmeldenummer:
**PCT/EP93/01735**

(87) Internationale Veröffentlichungsnummer:
**WO 94/00429 (06.01.1994 Gazette 1994/02)**

(54) **22-EN-25-OXA-DERIVATE IN DER VITAMIN D-REIHE, VERFAHREN ZU IHRER HERSTELLUNG, DIESE DERIVATE ENTHALTENEN PHARMAZEUTISCHE PRÄPARATE SOWIE DEREN VERWENDUNG ALS ARZNEIMITTEL**

22-ENE-25-OXA DERIVATIVES OF THE VITAMIN D SERIES, METHOD OF PREPARING SUCH DERIVATIVES, PHARMACEUTICAL PREPARATIONS CONTAINING THEM AND THUS USE OF SUCH PREPARATIONS AS DRUGS

DERIVES 22-EN-25-OXA DE LA SERIE DE LA VITAMINE D, LEUR PROCEDE DE PRODUCTION, PREPARATIONS PHARMACEUTIQUES CONTENANT CES DERIVES ET LEUR UTILISATION COMME MEDICAMENTS

(84) Benannte Vertragsstaaten:
**AT BE CH DE DK ES FR GB GR IE IT LI LU MC NL PT SE**

(30) Priorität: **30.06.1992 DE 4221961**

(43) Veröffentlichungstag der Anmeldung:
**26.04.1995 Patentblatt 1995/17**

(73) Patentinhaber: **SCHERING AKTIENGESELLSCHAFT**
**13342 Berlin (DE)**

(72) Erfinder:
- **STEINMEYER, Andreas**
  **D-14052 Berlin (DE)**
- **KIRSCH, Gerald**
  **D-14199 Berlin (DE)**
- **NEEF, Günter**
  **D-10711 Berlin (DE)**
- **SCHWARZ, Katica**
  **D-10585 Berlin (DE)**
- **THIEROFF-EKERDT, Ruth**
  **D-13469 Berlin (DE)**
- **WIESINGER, Herbert**
  **D-10781 Berlin (DE)**
- **HABEREY, Martin**
  **D-12247 Berlin (DE)**

(56) Entgegenhaltungen:
**EP-A- 0 441 467** **WO-A-89/10351**
**WO-A-89/10352**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft 22-En-25-oxa-Derivate in der Vitamin D-Reihe der allgemeinen Formel I

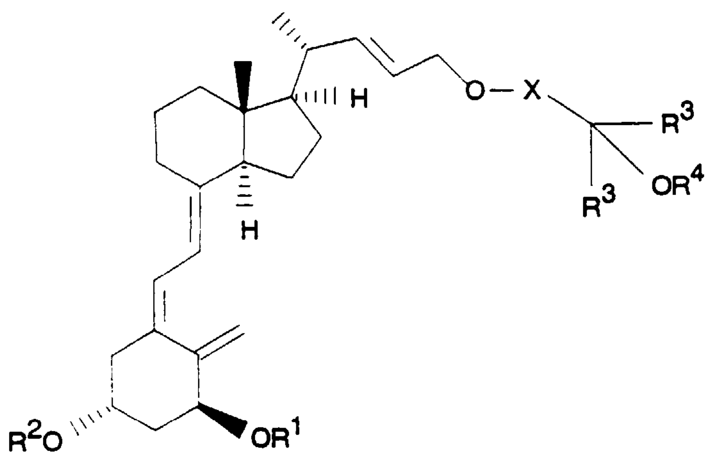

(I),

worin

| | |
|---|---|
| $R^1$, $R^2$ und $R^4$ | unabhängig voneinander je ein Wasserstoffatom, eine Alkanoylgruppe mit 1 bis 9 Kohlenstoffatomen oder eine Aroylgruppe, |
| $R^3$ | je ein Wasserstoffatom oder je eine lineare oder verzweigte Alkylgruppe mit 1 bis 4 Kohlenstoffatomen, je eine Trifluormethylgruppe oder einen gemeinsamen mit dem tertiären Kohlenstoffatom gebildeten gesättigten oder ungesättigten carbocyclischen oder heterocyclischen 3, 4, 5 oder 6-gliedrigen Ring und |
| X | einen Alkylenrest $-(CH_2)_n-$ mit n=1, 2 oder 3 |

bedeuten,
ein Verfahren zu deren Herstellung, pharmazeutische Präparate, die diese Verbindungen enthalten sowie deren Verwendung zur Herstellung von Arzneimitteln.

Die für die Reste $R^1$, $R^2$ und $R^4$ möglichen Alkanoylgruppen sind insbesondere von gesättigten Alkancarbonsäuren und die Aroylgruppe von der Benzoesäure abgeleitet.

Als Alkylgruppen für $R^3$ kommen in erster Linie die Methyl-, Ethyl- oder Propylgruppe oder ein gemeinsam mit dem tertiären Kohlenstoffatom gebildeter Cyclopropyl- oder Cyclopentylring infrage.

Bevorzugt gemäß vorliegender Erfindung sind 22-En-25-oxa-Verbindungen der allgemeinen Formel I, in welcher $R^1$, $R^2$ und $R^4$ für ein Wasserstoffatom und $R^3$ für je eine Methyl-, Ethyl- oder Propylgruppe sowie X für $CH_2$ stehen. Insbesondere bevorzugt sind die Verbindungen:

(5Z,7E,22E)-(1S,3R)-24-(2-Hydroxy-2-methylpropoxy)-9,10-secochola-5,7,10(19),22-tetraen-1,3-diol,
(5Z,7E,22E)-(1S,3R)-24-(2-Ethyl-2-hydroxybutoxy)-9,10-secochola-5,7,10(19),22-tetraen-1,3-diol,
(5Z,7E,22E)-(1S,3R)-24-(2-Hydroxy-2-propylpentoxy)-9,10-secochola-5,7,10(19),22-tetraen-1,3-diol.
22-En-26-oxa-Derivate in der Vitamin D-Reihe sind bereits beschrieben worden (Schering AG, PCT-Anmeldung WO 91/12238).

Die natürlichen Vitamine $D_2$ und $D_3$ (vergl. allg. Formel XV) sind an sich biologisch inaktiv und werden erst nach Hydroxylierung in 25-Position in der Leber bzw. in 1-Position in der Niere in deren biologisch aktive Metaboliten umgewandelt. Die Wirkung der Vitamine $D_2$ und $D_3$ besteht in der Stabilisierung des Plasma-$Ca^{++}$- und Plasma-Phosphat-Spiegels; sie wirken einem Absinken des Plasma-Ca++-Spiegel entgegen.

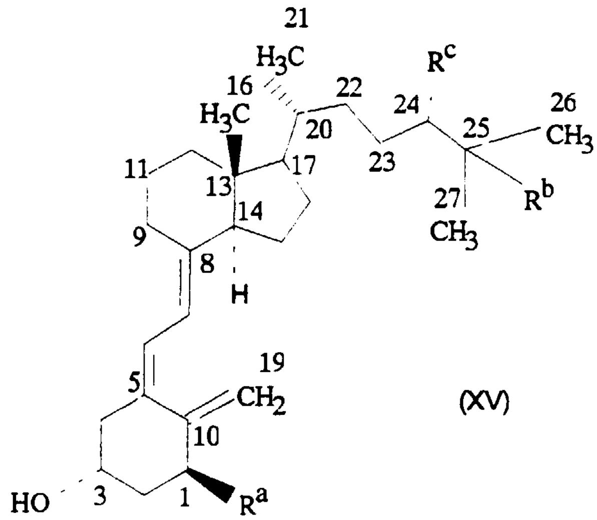

(XV)

Ergocalciferol: $R^a = R^b = H$, $R^c = CH_3$ Vitamin $D_2$
Doppelbindung C-22/23
Cholecalciferol: $R^a = R^b = R^c = H$ Vitamin $D_3$
25-Hydroxycholecalciferol: $R^a = R^c = H$, $R^b = OH$
1α-Hydroxycholecalciferol: $R^a = OH$, $R^b = R^c = H$
1α,25-Dihydroxycholecalciferol $R^a = R^b = OH$, $R^c = H$ Calcitriol

Neben ihrer ausgeprägten Wirkung auf den Calcium- und Phosphatstoffwechsel besitzen Vitamin $D_2$ und $D_3$ und seine synthetischen Abkömmlinge auch proliferationshemmende und zelldifferenzierende Wirkungen (H.F. De Luca, The Metabolism and Function of Vitamin D in Biochemistry of Steroid Hormones. Hrsg. H.L.J. Makin, 2nd Edition. Blackwell Scientific Publications 1984, S. 71-116).

Bei Vitamin D-Anwendung kann es aber zu Überdosierungserscheinungen kommen (Hypercalcämie).

In 24-Stellung hydroxylierte 1α-Cholecalciferole gehen bereits aus der DE-AS 25 26 981 hervor; sie besitzen eine geringere Toxizität als das entsprechende nicht-hydroxylierte 1α-Cholecalciferol. Die hydroxylierten Verbindungen zeigen eine selektive Aktivierung der intestinalen Calciumabsorption und eine schwächere Knochenabsorptionswirkung als 1α-Cholecalciferol.

Die in der internationalen Patentanmeldung WO 87/00834 beschriebenen 24-Hydroxy-Vitamin-D-Analoga können für die Behandlung von durch abnorme Zellproliferation und/oder Zelldifferentiation hervorgerufenen Störungen beim Menschen und Tier dienen.

Die in den Entgegenhaltungen WO-A-89/10351, WO-A-89/10352 und EP-A-0441467 beschriebenen calcitriolwirksamen Verbindungen besitzen keine 25-Oxa-Struktur.

Für verschiedene 1,25-Dihydroxy-Homo-Vitamin-D-Derivate ist eine Dissoziation bezüglich der Eigenschaften Knochenabsorptionswirkung und HL-60 Zelldifferentiation schon kürzlich von De Luca erwähnt worden. Die Knochenabsorptionswirkung in vitro ist dabei ein direktes Maß für die Calciummobilisierung in vivo.

Die Vitamin D-Aktivität der erfindungsgemäßen Verbindungen wird mittels eines Calcitriol-Rezeptortests bestimmt. Die Rezeptorpräparation wird aus Schweinedarmmucosa erhalten (M.C. Dame, E.A. Pierce, H.F. DeLuca; Proc. Natl. Acad. Sci. USA **82**, 7825 (1985)). Rezeptorhaltiges Bindungsprotein wird mit Calcitriol (0,025 µCi) in einem Reaktionsvolumen von 0.25 ml in Abwesenheit und in Anwesenheit der Prüfsubstanz für 2 Stunden bei 4°C in einem Teströhrchen inkubiert. Zur Trennung von freiem und rezeptorgebundenem Calcitriol wird eine Charcoal-Dextran-absorption durchgeführt. Dazu werden 250 µl einer Charcoal-Dextran-Suspension jedem Teströhrchen zugeführt, 20 Minuten inkubiert und bei 4°C zentrifugiert. Der Überstand wird dekantiert und nach einstündiger Äquilibrierung in Atom-Light in einem ß-Zähler gemessen.

Die mit verschiedenen Konzentrationen der Prüfsubstanz sowie der Referenzsubstanz (unmarkiertes Calcitriol) bei konstanter Konzentration der Bezugssubstanz ($^3$H-Calcitriol) erhaltenen Kompetitionskurven werden in Beziehung zueinander gesetzt und ein Kompetitionsfaktor (KF) ermittelt.

Er ist definiert als Quotient aus den Konzentrationen der jeweiligen Prüfsubstanz und der Referenzsubstanz, die für 50%ige Kompetition erforderlich sind:

$$KF = \frac{\text{Konzentration Prüfsubstanz bei 50\% Kompetition}}{\text{Konzentration Referenzsubstanz bei 50\% Kompetition}}$$

Demnach besitzen (5Z,7E,22E)-(1S,3R)-24-(2-Hydroxy-2-methylpropoxy)-9,10-secochola-5,7,10(19),22-tetraen-1,3-diol (16) einen $K_F$ von 4,4 und (5Z,7E,22E)-(1S,3R)-24-(2-Ethyl-2-hydroxybutoxy)-9,10-secochola-5,7,10(19),22-tetraen-1,3-diol (17) einen $K_F$ von 2,0. Beide Verbindungen binden also etwa gleich stark wir Calcitriol ($K_F$ = 1) an den Vitamin D-Rezeptor.

Es ist literaturbekannt (D.J. Mangelsdorf et al, J. Cell. Biol. **98**: 391-398 (1984)), daß die Behandlung humaner Leukämiezellen (Promyelozytenzellinie HL 60) in vitro mit Calcitriol die Differenzierung der Zellen zu Makrophagen induziert.

Zur Quantifizierung der differenzierungsstimulierenden Wirkung von Calcitriolanaloga wird der nachfolgend aufgeführte Test durchgeführt:

HL 60-Zellen werden in Gewebekulturmedium (RPMI-10% fetales Kälberserum) bei 37°C in einer Atmosphäre 5% $CO_2$ in Luft kultiviert.

Zur Substanztestung werden die Zellen abzentrifugiert und 2.8 x $10^5$ Zellen/ml in phenolrotfreiem Gewebekulturmedium aufgenommen. Die Testsubstanzen werden in Ethanol gelöst und mit Gewebekulturmedium ohne Phenolrot auf die gewünschte Konzentration verdünnt. Die Verdünnungsstufen werden mit der Zellsuspension in einem Verhältnis von 1:10 gemischt und je 100µl dieser mit Substanz versetzten Zellsuspension in eine Vertiefung einer 96-Loch-Platte pipetiert. Zur Kontrolle wird eine Zellsuspension analog mit dem Lösungsmittel versetzt.

Nach Inkubation über 96 Stunden bei 37°C in 5% $CO_2$ in Luft wird jede Vertiefung der 96-Loch-Platte zu der Zellsuspension 100µl einer NBT-TPA-Lösung (Nitroblautetrazolium (NBT), Endkonzentration im Ansatz 1 mg/ml, Tetradecanoylphorbolmyristat-13-acetat (TPA), Endkonzentration im Ansatz 2 x $10^{-7}$ mol/l) pipettiert.

Durch Inkubation über 2 Stunden bei 37°C und 5% $CO_2$ in Luft wird infolge der intrazellulären Sauerstoffradikalfreisetzung, stimuliert durch TPA, in den zu Makrophagen differenzierten Zellen NBT zu unlöslichem Formazan reduziert.

Zur Beendigung der Reaktion werden die Vertiefungen der 96-Loch-Platte abgesaugt und die anhaftenden Zellen durch Zugabe von Methanol fixiert und nach Fixation getrocknet.

Zur Lösung der gebildeten intrazellulären Formazankristalle werden in jede Vertiefung 100 µl Kaliumhydrioxid (2 val/l) und 100 µl Dimethylsulfoxid pipettiert und 1 Minute ultrabeschallt. Die Konzentration von Formazan wird spektralphotometrisch bei 650 nm gemessen.

Als Maß für die Differenzierungsinduktion der HL 60-Zellen zu Makrophagen gilt die Konzentration an gebildetem Formazan. Die relative Wirksamkeit der Testsubstanz ergibt sich aus dem Quotienten $ED_{50}$ Testsubstanz/$ED_{50}$ Calcitriol.

Demnach besitzen Calcitriol, (5Z,7E,22E)-(1S,3R)-24-(2-Hydroxy-2-methylpropoxy)-9,10-secochola-5,7,10(19),22-tetraen-1,3-diol (16) und (5Z,7E,22E)-(1S,3R)-24-(2-Ethyl-2-hydroxybutoxy)-9,10-secochola-5,7,10(19),22-tetraen-1,3-diol (17) die $ED_{50}$-Werte 1,6 x $10^{-9}$ mol/l, 3,2 x $10^{-9}$ mol/l und 1,0 x $10^{-9}$ mol/l. Die erfindungsgemäßen Substanzen unterscheiden sich in ihrer differenzierungsinduzierenden Wirkung praktisch nicht von Calcitriol.

Calcitriol besitzt eine regulatorische Wirkung auf den Calcium-Metabolismus. Zur Abschätzung der entsprechenden Aktivitäten der erfindungsgemäßen Substanzen werden die hyper-calcämische und hypercalciurische Aktivität in vivo bestimmt.

Calcitriol (2 µg/kg) und die Testsubstanzen (jeweils 200 µg/kg) werden in 500 µl Ethanol/0,9% NaCl-Lösung (40: 60 v/v) subcutan intakten männlichen Wistar-Ratten (140-160 g), die normal ernährt werden (Altromin™, Wasser), verabreicht. Im Zeitraum von 0-16 Stunden nach Substanzapplikation wird Urin gesammelt. Anschließend erhalten die Tiere oral 1 µmol Calcium in 6,5% Klucell (Hydroxypropylcellulose), und erneut wird Urin für den Zeitraum 16-22 Stunden nach Substanzapplikation gesammelt. Das Urinvolumen jeder Fraktion wird gemessen, um die absolute Calciumexkretion zu messen.

Nach 22 Stunden werden die Tiere durch Dekapitation getötet und das Blut gesammelt. Die Calciumkonzentration in Serum und Urin wird durch Flammenphotometrie bestimmt.

Anhand der gemessenen Daten ist im Vergleich zu Calcitriol die in vivo-Potenz von (5Z,7E,22E)-(1S,3R)-24-(2-Hydroxy-2-methylpropoxy)-9,10-secochola-5,7,10(19),22-tetraen-1,3-diol (16) um mehr als den Faktor 100, von (5Z,7E,22E)-(1S,3R)-24-(2-Ethyl-2-hydroxybutoxy)-9,10-secochola-5,7,10(19),22-tetraen-1,3-diol (17) um etwa den Faktor 100 reduziert.

In einer Dosierung von 200 µg/kg induziert 16 keine, 17 eine leichte Hypercalcämie, die mit dem Anstieg der Calciumkonzentration bei einer Calcitrioldosis von 2 µg/kg vergleichbar war (Serumcalciumkonzentration: Lösungsmittelkontrolle 3,0 mmol/l, Calcitriol 3,2 mmol/l, 16 3,0 mmol/l, 17 3,4 mmol/l). Die Calciumexkretion war in der ersten Sammelphase (0-16 Stunden) jeweils leicht erhöht gegenüber der Lösungsmittelkontrolle aber etwa identisch mit der Calciumexkretion, die durch Calcitriol in 100-fach niedrigerer Dosierung ausgelöst wurde: Calciumkonzentration (1. Sammelphase): Lösungsmittelkontrolle 12,2 µmol/Tier, Calcitriol 40,6 µmol/Tier, 16 62,4 µmol/Tier, 17 61,7 µmol/Tier.

In der 2. Sammelphase (16-22 Stunden) war nach Applikation von 17 (200 µg/kg) die Calciumexkretion nicht signifikant unterschiedlich zum Standard Calcitriol (2µg/kg); die Calciumexkretion nach Applikation von 16 (200 µg/kg) nicht erhöht gegenüber der Lösungsmittelkontrolle und somit signifikant niedriger als bei Calcitriol-Verabreichung: Calciumkonzentration (2. Sammelphase): Lösungsmittelkontrolle 1,5 µmol/Tier, Calcitriol 25,1 µmol/Tier, 16 3,6 µmol/Tier, 17 47,0 µmol/Tier. Diese Befunde sprechen für eine Wirkungsdissoziation zwischen wachstumsinhibierender und calcitroper Wirkung der Testsubstanzen bei systemischer Applikation im Vergleich zu Calcitriol.

Durch das verminderte Hypercalcämie-Risiko eignen sich die erfindungsgemäßen Substanzen in besonderer Weise zur Herstellung von Arzneimitteln für die Behandlung von Erkrankungen, die durch eine Hyperproliferation gekennzeichnet sind, z.B. hyperproliferative Erkrankungen der Haut (Psoriasis) und maligne Tumoren (Leukämie, Coloncarcinom, Mammacarcinom) und Akne (J. Invest. Dermatol., Vol. 92, No. 3, 1989).
In einer besonders bevorzugten Ausführungsform der vorliegenden Erfindung werden vor der Behandlung mit den erfindungsgemäßen Verbindungen Calcitriolrezeptoren im Zielorgan nachgewiesen.
Weiterhin wurde gefunden, daß durch topische Applikation der erfindungsgemäßen Verbindungen auf die Haut von Mäusen, Ratten und Meerschweinchen eine vermehrte Hautrötung und Zunahme der Epidermisdicke induziert werden kann. Die Zunahme der Hautrötung wird anhand der Erhöhung des mit einem Farbmeßgerät quantifizierbaren Rotwertes der Hautoberfläche ermittelt. Der Rotwert ist nach dreimaliger Substanzapplikation im Abstand von 24 Stunden deutlich erhöht. Die Zunahme der Epidermisdicke wird im histologischen Präparat quantifiziert und ist ebenfalls erhöht.
Diese Eigenschaften der erfindungsgemäßen Vitamin D-Verbindungen läßt sie zum therapeutischen Einsatz bei atrophischer Haut, wie sie bei natürlicher Hautalterung, vorzeitiger Hautalterung infolge erhöhter Lichtexposition oder medikamentös induzierter Hautatrophie durch Behandlung mit Glucocorticoiden auftritt, geeignet erscheinen.
Weiterhin kann die Wundheilung durch topische Applikation mit den neuen Verbindungen beschleunigt werden.
Die erfindungsgemäßen Verbindungen der allgemeinen Formel I sind auch potente Hemmstoffe der Proliferation und Interleukin (IL 2)-Synthese von humanen Lymphocyten. Infolge der Hemmung der Lymphocytenproliferation und IL 2-Synthese in niedriger Konzentration sind die erfindungsgemäßen Verbindungen der allgemeinen Formel I zur Therapie von Erkrankungen des Immunsystems geeignet, z. B. Erkrankungen des atopischen Formenkreises (atopische Dermatitis, Asthma), Autoimmunerkrankungen einschließlich Diabetes mellitus, Transplantatabstoßungsreaktionen und AIDS.
Für Calcitriol wurde gefunden, daß es aufgrund eines rezeptorvermittelten Mechanismus nicht nur die IL 2-Sekretion, sondern auch die Produktion anderer entzündungsfördernder Cytokine hemmt. Da die Verbindungen der allgemeinen Formel I etwa ebenso gut an den Rezeptor binden wie Calcitriol sind sie geeignet zur Behandlung von entzündlichen Erkrankungen wie Arthritis, Colitis ulcerosa und Morbus Crohn.
Bei der Behandlung von Autoimmunerkrankungen, Transplantatabstoßungsreaktionen und AIDS können die neuen Verbindungen der allgemeinen Formel I vorteilhaft mit anderen immunsuppressiv wirksamen Stoffen wie Cyclosporin A und FK 506 kombiniert werden.

Die vorliegende Erfindung bezieht sich somit auch auf pharmazeutische Präparate, die mindestens eine Verbindung der allgemeinen Formel I zusammen mit einem pharmazeutisch verträglichen Träger enthalten.
Die Verbindungen können formuliert werden als Lösungen in pharmazeutisch verträglichen Solventien oder als Emulsionen, Suspensionen oder Dispersionen in geeigneten pharmazeutischen Solventien oder Trägern oder als Pillen, Tabletten oder Kapseln, die in an sich bekannter Weise feste Trägerstoffe enthalten. Für eine topische Anwendung werden die Verbindungen vorteilhafterweise als Cremes oder Salben oder in einer ähnlichen, zur topischen Anwendung geeigneten Arzneimittelform formuliert. Jede derartige Formulierung kann auch andere pharmazeutisch verträgliche und nichttoxische Hilfsmittel enthalten, wie z.B. Stabilisatoren, Antioxidantien, Bindemittel, Farbstoffe, Emulgatoren oder Geschmackskorrigentien. Die Verbindungen werden vorteilhafterweise durch Injektion oder intravenöse Infusion geeigneter steriler Lösungen oder als orale Dosierung über den Ernährungstrakt oder topisch in der Form von Cremes, Salben, Lotionen oder geeigneter transdermaler Pflaster appliziert, wie in der EP-A-0387 077 beschrieben ist.

Die tägliche Dosis liegt bei 0,1 µg/Patient/Tag - 1000 µg(1 mg)/Patient/Tag, vorzugsweise 1,0 µg/Patient/Tag - 500 µg/Patient/Tag.

Die Verbindungen der allgemeinen Formel I, insbesondere auch die zu deren Herstellung benötigten Ausgangsverbindungen der allgemeinen Formel XIV, werden nach einem neuen Verfahren erhalten. Die Erfindung betrifft daher auch ein Verfahren zur Herstellung der Verbindungen der allgemeinen Formel I.

Die zur Herstellung der 22-En-25-oxa-Vitamin D-Derivate benötigten Ausgangsverbindungen der allgemeinen Formel XIV erfolgt auf einem konvergenten Syntheseweg, wobei der CD-Teil und der A-Teil separat aufgebaut werden.
Ausgangsmaterial für das CD-Fragment ist einer der literaturbekannten Aldehyde II (H.H. Inhoffen et al. Chem. Ber. **91**, 780 (1958), Chem. Ber. **92**, 1772 (1959), W.G. Dauben et al. Tetrahedron Lett. **30**, 677 (1989)),

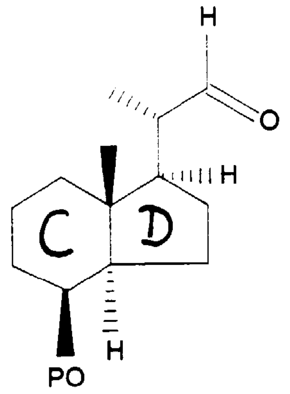

II

worin P eine acyl-, alkyl-, aryl- oder alkyl- und aryl (gemischt)-substituierte Silyl- oder Tetrahydropyranyl- (THP)-Gruppe bedeutet. Als Beispiele für die Silylgruppe P seien die tert.-Butyl-dimethylsilyl-, Trimethylsilyl-, tert.-ButylDurch Wadsworth-Emmons-Reaktion mit dem durch Deprotonierung mit einer Base (NaH, KH, Lithiumdiisopropylamid (LDA), Kalium-tert.-butylat) erzeugten Anion eines Phosphonates III

$$(RO)_2P(O)\text{-}CH_2\text{-}COOR' \qquad (III),$$

worin R und R' unabhängig voneinander geradkettig oder verzweigte Alkylgruppen mit bis zu 9 Kohlenstoffatomen oder Phenylgruppen bedeuten, erzeugt man eine Verbindung der allgemeinen Formel IV,

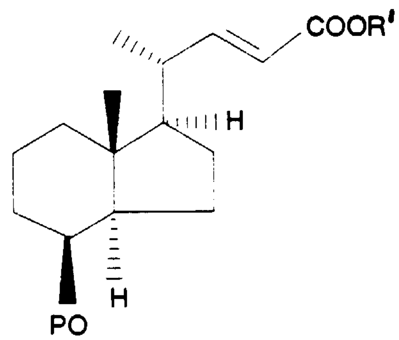

IV

deren Estergruppe mit einem Reduktionsmittel ($LiAlH_4$, Diisobutylaluminiumhydrid (DIBAH)) zum entsprechenden Alkohol der allgemeinen Formel V reduziert wird.

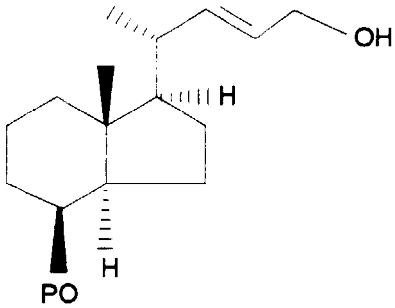

V

Durch Veretherung mit einer Verbindung der allgemeinen Formel VI

L-X-C(O)-OR" (VI),

worin L für eine Abgangsgruppe wie Br, I, $CH_3$-$C_6H_4$-$SO_2O$,
X für einen Alkenylrest $-(CH_2)_n-$ mit n = 1, 2 oder 3
sowie R" für einen geradkettigen oder verzweigten Alkylrest mit 1 bis 8 Kohlenstoffatomen stehen,
erhält man eine Verbindung der allgemeinen Formel VII.

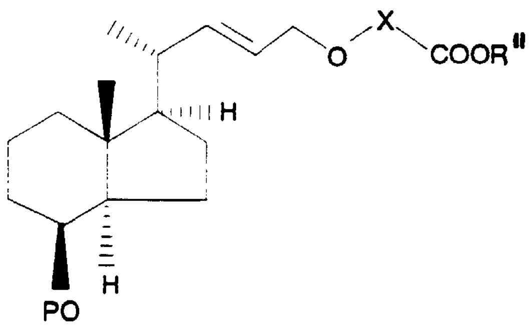

VII

An deren Estergruppe wird ein nukleophiles Reagenz der allgemeinen Formel VIII

$R^3$-M (VIII)

addiert (mindestens einfacher Überschuß), worin $R^3$ eine lineare oder verzweigte Alkylgruppe mit 1 bis 4 Kohlenstoffatomen und M MgHal (Hal = Cl, Br, I) oder ein Alkaliatom (Li, Na, K) bedeuten,
wobei man eine Verbindung der allgemeinen Formel IX erhält.

Sollen für $R^3$ in der letztendlich gewünschten Verbindung der allgemeinen Formel I zwei Wasserstoffatome stehen, wird anstelle von $R^3$-M (VIII) ein hydrid-lieferndes Reagenz, z.B. Lithiumaluminiumhydrid, mit VII umgesetzt.

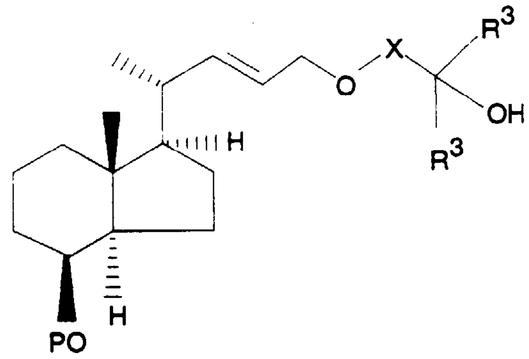

IX

Die Schutzgruppe P in IX wird abgespalten. Im Falle der Acylgruppe verwendet man basische Bedingungen ($K_2CO_3$, Methanol; KOH oder NaOH, Methanol), im Falle einer Silylschutzgruppe verwendet man Fluorid-Reagenzien (Tetrabutylammoniumfluorid, HF, HF/Pyridin) und im Falle der THP-Schutzgruppe verwendet man Säurekatalyse (p-Toluolsulfonsäure, Pyridinium-p-toluolsulfonat, Ionentauscher), so daß eine Verbindung der allgemeinen Formel X entsteht.

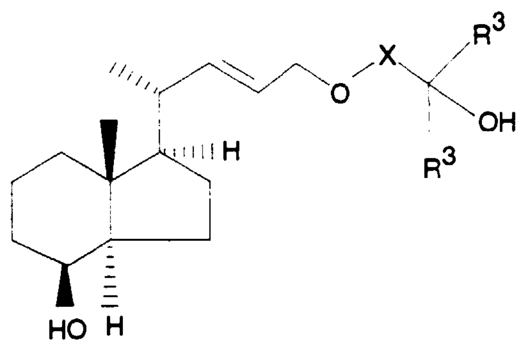

X

Mit einem Oxidationsmittel wie Pyridiniumchlorochromat (PCC), Pyridiniumdichromat (PDC), Collins-Reagenz oder $BaMnO_4$ wird die sekundäre Hydroxylgruppe zum Keton oxidiert, wobei eine Verbindung der allgemeinen Formel XI entsteht.

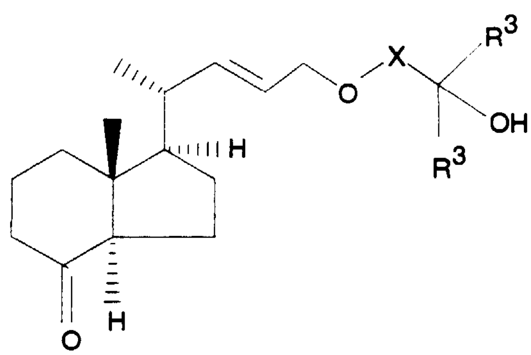

XI

Die tertiäre Hydroxylgruppe in XI wird mit einer Hydroxyschutzgruppe Z, beispielsweise der Trimethylsilyl-, Dimethyl-tert.-butylsilyl-, einer alkyl-aryl-substituierten Silylgruppe, dem dem Tetrahydropyranyl- oder Tetrahydrofuranylrest, geschützt, wobei eine Verbindung der allgemeinen Formel XII entsteht.

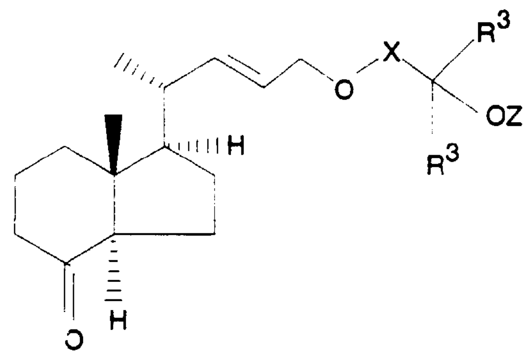

XII

Durch Horner-Wittig-Reaktion mit dem durch eine Base wie n-Butyllithium oder Lithiumdiisopropylamid (BuLi, LDA) erzeugten Anion des literaturbekannten Phoshinoxids XIII (M.R. Uskokovic J. Org. Chem. **51**, 3098 (1986))

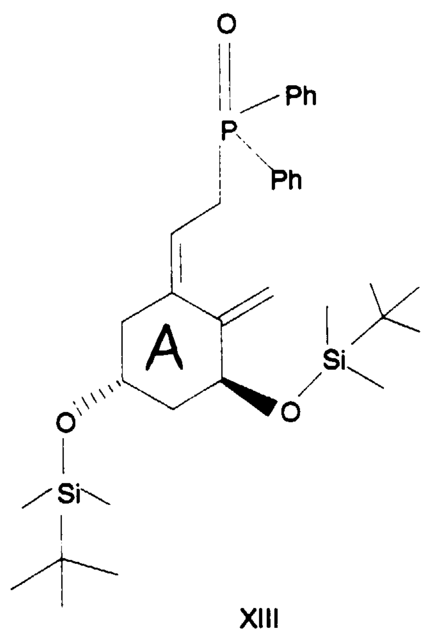

XIII

erhält man eine Verbindung der allgemeinen Formel XIV,

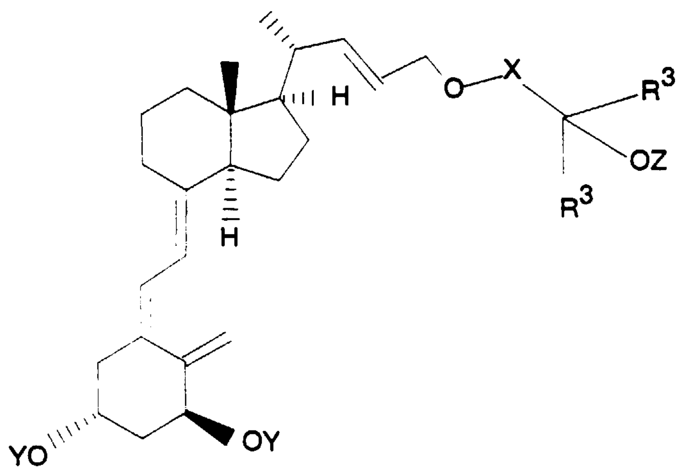

(XIV),

worin Y jeweils eine dimethyl, t-butyl-Silyl-Hydroxyschutzgruppe und Z eine Silyl-, Tetrahydropyranyl- oder Tetrahydrofuranyl-Gruppe als Schutzgruppe bedeuten.

Durch Abspaltung der Schutzgruppen wird die Verbindung der allgemeinen Formel XIV in eine Verbindung der allgemeinen Formel I, worin $R^1$, $R^2$ und $R^4$ Wasserstoffatome bedeuten, und diese anschließend gewünschtenfalls durch Veresterung der freien Hydroxygruppen in eine Verbindung der allgemeinen Formel I, worin $R^1$, $R^2$ und $R^4$ $C_1$-$C_9$-Alkanoyl- oder Benzoylgruppen bedeuten, überführt.

Die Abspaltung der Hydroxyschutzgruppen erfolgt vorzugsweise unter Verwendung von Tetra-n-butyl-ammoniumfluorid, HF oder HF-Pyridinkomplex.

Die mögliche Veresterung freier Hydroxygruppen erfolgt nach gängigen Verfahren partiell, sequentiell oder vollständig mit dem entsprechenden Carbonsäurehalogenid (Halogenid = Chlorid, Bromid) oder Carbonsäureanhydrid.

Die nachfolgenden Beispiele dienen der näheren Erläuterung der Erfindung:

**Beispiel 1**

[1R-[1α[R*-(E)],3aβ,4α,7aα]]-4-[4-[[Dimethyl(1,1-dimethylethyl)silyl]oxy]-7a-methyl-octahydro-1H-inden-1-yl]-2-pentensäuremethylester <u>2</u>

Man legt 1,55 g (38,7 mmol) Natriumhydrid (60%) in 60 ml Tetrahydrofuran unter Argon vor und tropft bei Raumtemperatur langsam 7,0 g (38,7 mmol) Dimethyl(methoxycarbonyl)-methylphosphonat in 60 ml Tetrahydrofuran zu. Nach 30 Minuten bei Raumtemperatur tropft man 4,1 g (12,6 mmol) [1R-[1α(S*),3aβ,4α,7aα]-α,7a-Dimethyl-4-[[dimethyl(1,1-dimethylethyl)silyl]oxy]octahydro-1H-inden-1-acetaldehyd <u>1</u> (W.G. Dauben et al. Tetrahedron Lett. <u>30</u> 677 (1989)) in 80 ml Tetrahydrofuran zu und rührt über Nacht nach. Unter Eiskühlung wird nun gesättigte Natriumchlorid-Lösung zugetropft, mit Essigester extrahiert, die organische Phase mit Natriumchlorid-Lösung gewaschen, über Natriumsulfat getrocknet und das Solvens entfernt. Man reinigt den Rückstand chromatographisch an Kieselgel mit Hexan/Essigester als Elutionsmittel, wobei 3,6 g der Titelverbindung als farbloses Öl anfallen.

$^1$H-NMR ($CDCl_3$): δ = 0,01 und 0,03 ppm (2x s, je 3H, Si-$Me_2$); 0,92 (s, 9H, Si-t-butyl); 0,98 (s,3H,H-18); 1,08 (d,J=7Hz, 3H,H-21); 3,72 (s,3H,COOMe); 4,01 (m,1H,H-8); 5,75 (d,J=15Hz,1H,H-23); 6,85 (dd,J=15,9Hz,1H,H-22) [Es wird durchgängig die Steroid-Nummerierung verwendet]

IR (Film): ν=1720 $cm^{-1}$

**Beispiel 2**

[1R-[1α[R*-(E)],3aβ,4α,7aα]]-4-[4-[[Dimethyl(1,1-dimethylethyl)silyl]oxy]-7a-methyl-octahydro-lH-inden-1-yl]-2-penten-1-ol <u>3</u>

Man legt 3,6 g (9,46 mmol) <u>2</u> in 150 ml Tetrahydrofuran vor und tropft unter Argon bei 0°C 37,8 ml DIBAH (1M in Hexan) zu. Es wird 90 Minuten bei 0°C gerührt, Natriumchlorid-Lösung zugegeben, mit Essigester extrahiert, die organische Phase mit Natriumchlorid-Lösung gewaschen, über Natriumsulfat getrocknet und eingeengt. Der Rückstand wird an Kieselgel mit Hexan/Essigester gereinigt, wobei man 3,0 g der Titelverbindung als farbloses Öl erhält.

$^1$H-NMR ($CDCl_3$): δ = 0,00 und 0,01 ppm (2x s, je 3H, Si-$Me_2$); 0,88 (s, 9H, Si-t-butyl); 0,95 (s,3H,H-18); 1,02 (d,J=7Hz, 3H,H-21); 4,00 (m,1H,H-8); 4,08 (sbr,2H,H-24); 5,55 (m,2H,H-22 u. H-23)

**Beispiel 3**

[1R-[1α[R*-(E)],3aβ,4α,7aα]]-[[4-[4-[[Dimethyl(1,1-dimethylethyl)silyl]oxy]-7a-methyl-octahydro-1H-inden-1-yl]-2-pentenyl]oxy]essigsäure-1,1-dimethylethylester 4

Man löst 3,0 g (8,5 mmol) 3 in 3,5 ml Toluol und gibt unter Argon 39 ml wäßrige Natriumhydroxid-Lösung (25%), 545 mg Tetrabutylammoniumhydrogensulfat und 12,08 g (61,9 mmol) Bromessigsäure-tert.-butylester zu. Es wird über Nacht bei Raumtemperatur gerührt und anschließend mit Natriumchlorid-Lösung versetzt. Nach Extraktion mit Essigester, Waschen der organischen Phase mit Natriumchlorid-Lösung, Trocknen über Natriumsulfat und Entfernen des Lösungsmittels, reinigt man den Rückstand chromatographisch an Kieselgel mit Hexan/Essigester, wobei 2,3 g der Titelverbindung als farbloses Öl verbleiben.

$^1$H-NMR ($CDCl_3$): δ = 0,00 und 0,02 ppm (2x s, je 3H, Si-$Me_2$); 0,90 (s, 9H, Si-t-butyl); 0,96 (s,3H,H-18); 1,02 (d, J=7Hz,3H,H-21); 1,50 (s,9H,COO-t-butyl); 3,94 (s,2H,H-26); 4,01 (m,1H,H-8); 4,02 (d,J=5,5Hz,2H,H-24); 5,47 (dt, J=15,5, 5,5Hz,1H,H-23); 5,58 (dd,J=15,5, 9Hz,1H,H-22)

**Beispiel 4**

[1R-[1α[R*-(E)],3aβ,4α,7aα]]-1-[[4-[4-[[Dimethyl(1,1-dimethylethyl)silyl]oxy]-7a-methyloctahydro-1H-inden-1-yl]-2-pentenyl]oxy]-2-methyl-2-propanol 5

Aus 299 mg (12,3 mmol) Magnesiumspänen und 1,75 g (12,3 mmol) Jodmethan in 10 ml Ether wird das Grignard-Reagenz bereitet. Bei 0°C tropft man nun 1,15 g (2,46 mmol) 4 in 30 ml Ether unter Argon zu. Es wird 2 Stunden bei Raumtemperatur gerührt und anschließend mit Ammoniumchlorid-Lösung hydrolysiert. Nach Extraktion mit Essigester, Waschen der organischen Phase mit Natriumchlorid-Lösung, Trocknen über Natriumsulfat und Entfernen des Lösungsmittels chromatographiert man das Rohprodukt an Kieselgel mit Hexan/Essigester, wobei 946 mg der Titelverbindung als farbloses Öl anfallen.

$^1$H-NMR ($CDCl_3$): δ = 0,00 und 0,01 ppm (2x s, je 3H, Si-$Me_2$); 0,90 (s, 9H, Si-t-butyl); 0,94 (s,3H,H-18); 1,02 (d, J=7Hz,3H,H-21); 1,22 (s,6H,H-28 u. H-29)); 3,23 (m,2H,H-26); 3,95 (dd,J=12,5, 5,5Hz,1H,H-24); 4,00 (dd,J=12,5, 5,5Hz,1H,H-24'); 4,00 (m,lH,H-8); 5,42 (dt,J=15,5, 5,5Hz,1H,H-23); 5,53 (dd,J=15,5, 9Hz,1H,H-22)

**Beispiel 5**

[1R-[1α[R*-(E)],3aβ,4α,7aα]]-3-[[[4-[4-[[Dimethyl(1,1-dimethylethyl)silyl]oxy]-7a-methyloctahydro-1H-inden-1-yl]-2-pentenyl]oxy]methyl-3-pentanol 6

Aus 1,34 g (12,3 mmol) Bromethan und 299 mg (12,3 mmol) Magnesiumspänen wird in 10 ml Tetrahydrofuran das Grignard-Reagenz bereitet und analog Beispiel 4 mit 4 umgesetzt. Man erhält nach Aufreinigung 1,09 g der Titelverbindung als farbloses Öl.

$^1$H-NMR ($CDCl_3$): δ = 0,00 und 0,01 ppm (2x s, je 3H, Si-$Me_2$); 0,89 (t,J=7Hz,6H,H-30 u. H-31); 0,90 (s, 9H, Si-t-butyl); 0,94 (s,3H,H-18); 1,02 (d,J=7Hz,3H,H-21); 1,51 (q,J=7Hz, 4H, H-28 u. H-29); 3,27 (m,2H,H-26); 3,92 (dd,J=12,5, 5,5Hz,1H,H-24); 3,98 (dd,J=12,5, 5,5Hz,1H,H-24'); 4,00 (m,1H,H-8); 5,42 (dt,J=15,5, 5,5Hz,1H,H-23); 5,53 (dd, J=15,5, 9Hz,1H,H-22)

**Beispiel 6**

[1R-[1α[R*-(E)],3aβ,4α,7aα]]-1-[4-(2-Hydroxy-2-methylpropoxy)-1-methyl-2-butenyl]-7a-methyloctahydro-lH-inden-4-ol 7

Man löst 946 mg (2,22 mmol) 5 in 30 ml Tetrahydrofuran unter Argon und gibt 5,9 ml Fluorwasserstoffsäure/Pyridin-Komplex (70% HF) zu. Es wird 48 Stunden bei Raumtemperatur gerührt und anschließend mit 1 n Natronlauge neutralisiert. Nach Extraktion mit Essigester, Waschen der organischen Phase mit Natriumchlorid-Lösung, Trocknen über Natriumsulfat und Entfernen des Lösungsmittels reinigt man den Rückstand an Kieselgel mit Hexan/Essigester, wobei man 530 mg der Titelverbindung als farbloses Öl erhält.

$^1$H-NMR ($CD_2Cl_2$): δ = 0,90 ppm (s, 3H, H-18); 0,96 (d,J=7Hz,3H,H-21); 1,13 (s,6H, H-28 u.H-29); 3,16 (m,2H,H-26); 3,87, (dd,J=12,5, 5,5Hz,1H,H-24); 3,92 (dd,J=12,5, 5,5Hz,1H, H-24'); 3,98 (m,1H,H-8); 5,39 (dt,J=15,5, 5,5Hz, 1H,H-23); 5,47 (dd,J=15,5, 9Hz,1H,H-22)

**Beispiel 7**

[1R-[1α[R*-(E)],3aβ,4α,7aα]]-1-[4-(2-Ethyl-2-hydroxybutoxy)-1-methyl-2-butenyl]-7a-methyloctahydro-1H-inden-4-ol 8

Analog Beispiel 6 werden 909 mg (2,01 mmol) 6 in 30 ml Tetrahydrofuran mit 5,2 ml Fluorwasserstoffsäure/Pyridin-Komplex umgesetzt, wobei man 516 mg der Titelverbindung als farbloses Öl erhält.

$^1$H-NMR ($CDCl_3$): δ = 0,80 ppm (t,J=7Hz,6H,H-30 u. H-31); 0,90 (s, 3H,H-18); 0,96 (d,J=7Hz,3H,H-21); 1,43 (q, J=7Hz,4H, H-28 u.H-29)); 3,18 (m,2H,H-26); 3,85 (dd,J=12,5, 5,5Hz,1H,H-24); 3,90 (dd,J=12,5, 5,5Hz,1H,H-24'); 4,00 (m,IH,H-8); 5,38 (dt,J=15,5, 5,5Hz,1H,H-23); 5,46 (dd,J=15,5, 9Hz,1H,H-22)

**Beispiel 8**

[1R-[1α[R*-(E)],3aβ,4α,7aα]]-1-[4-(2-Hydroxy-2-propylpentoxy)-1-methyl-2-butenyl]-7a-methyloctahydro-1H-inden-4-ol 9

Aus 6,15 g (50 mmol) 1-Brompropan und 1,20 g (50 mmol) Magnesiumspänen wird in 50 ml Tetrahydrofuran das Grignard-Reagenz bereitet und analog Beispiel 4 mit 4 umgesetzt. Das Rohprodukt löst man erneut in 60 ml Tetrahydrofuran und setzt analog Beispiel 6 mit 12,4 ml Fluorwasserstoffsäure/Pyridin um, wobei man 1,13 g der Titelverbindung als farbloses Öl erhält.

$^1$H-NMR ($CDCl_3$): δ= 0,85 ppm (t, J=7 Hz, 6H, H-32 u. H-33); 0,89 (s, 3H, H-18); 0,95 (d, J=7 Hz, 3H, H-21); 3,14 (d, J=10 Hz, 1H, H-26); 3,19 (d, J=10 Hz, 1H, H-26'); 3,83 (dd, J=12,5, 5,5 Hz, 1H, H-24); 3,89 (dd, J=12,5, 7,5 Hz, 1H, H-24'); 4,00 (m, 1H, H-8); 5,36 (dt, J=15,5, 6 Hz, 1H, H-23); 5,45 (dd, J=15,5, 9 Hz, 1H, H-22)

**Beispiel 9**

[1R-[1α[R*-(E)],3aβ,7aα]]-1-[4-(2-Hydroxy-2-methylpropoxy)-1-methyl-2-butenyl]-7a-methyloctahydro-4H-inden-4-on 10

Man löst 530 mg (1,71 mmol) 7 in 40 ml Methylenchlorid unter Argon, gibt 538 mg (2,5 mmol) Pyridiniumchlorochromat zu und rührt 90 Minuten bei Raumtemperatur. Anschließend wird mit Ether verdünnt, mehrmals über Celite filtriert und das Solvens entfernt. Man chromatographiert das Rohprodukt an Kieselgel mit Hexan/Essigester, wobei 446 mg der Titelverbindung als farbloses Öl verbleiben.

$^1$H-NMR ($CDCl_3$): δ = 0,60 ppm (s, 3H,H-18); 1,01 (d,J=7Hz,3H,H-21); 1,13 (s,6H H-28 u.H-29); 2,40 (dd,J=10,5, 7Hz,1H,H-14); 3,16 (m,2H,H-26); 3,88 (dd,J=12,5, 5,5Hz,1H,H-24); 3,92 (dd,J=12,5, 5,5Hz,1H,H-24'); 5,40 (dt,J=15,5, 5,5Hz,1H,H-23); 5,49 (dd,J=15,5, 9Hz,1H,H-22)

**Beispiel 10**

[1R-[1α[R*-(E)],3aβ,7aα]]-1-[4-(2-Ethyl-2-hydroxybutoxy)-1-methyl-2-butenyl]-7a-methyloctahydro-4H-inden-4-on 11

Analog Beispiel 9 werden 516 mg (1,52 mmol) 8 mit 478 mg (2,22 mmol) Pyridiniumchlorochromat umgesetzt, wobei man 431 mg der Titelverbindung als farbloses Öl erhält.

$^1$H-NMR ($CDCl_3$): δ = 0,60 ppm (s, 3H,H-18); 0,80 (t,J=7Hz,6H,H-30); 1,01 (d,J=7Hz,3H,H-21); 1,45 (q,J=7Hz, H-28 u.H-29); 2,40 (dd,J=10,5, 7Hz,1H,H-14); 3,19 (m,2H,H-26); 3,85 (dd,J=12,5, 5,5Hz,1H,H-24); 3,90 (dd,J=12,5, 5,5Hz,1H,H-24'); 5,39 (dt,J=15,5, 5,5Hz,1H,H-23); 5,48 (dd,J=15,5, 9Hz,1H,H-22)

**Beispiel 11**

[1R-[1α[R*-(E)],3aβ,7aα]]-1-[4-(2-Hydroxy-2-propylpentoxy)-1-methyl-2-butenyl]-7a-methyloctahydro-4H-inden-4-on 12

Analog Beispiel 9 werden 1,13 g (3,08 mmol) 9 mit 966 mg (4,49 mmol) Pyridiniumchlorochromat umgesetzt, wobei man 958 mg der Titelverbindung als farbloses Öl erhält.

$^1$H-NMR ($CDCl_3$): δ= 0,60 ppm (s, 3H, H-18); 0,84 (t, J=7 Hz, 6H, H-32 u. H-33); 1,01 (d, J=7 Hz, 3H, H-21); 2,40 (dd, J=10,5, 7,5 Hz, 1H, H-14); 3,15 (d, J=10 Hz, 1H, H-26); 3,19 (d, J=10 Hz, 1H, H-26'); 3,84 (dd, J=12,2, 5,5 Hz, 1H, H-24); 3,89 (dd, J=12,5, 5,5 Hz, 1H, H-24'); 5,39 (dt, J=15,5, 6 Hz, 1H, 23); 5,48 (dd, J=15,5, 9 Hz, 1H, H-22)

**Beispiel 12**

[1R-[1α[R*-(E)],3aβ,7aα]]-7a-Methyl-1-[1-methyl-4-[2-methyl-2-[(trimethylsilyl)-oxy]propoxy]-2-butenyl]octahydro-4H-inden-4-on 13

Man löst 440 mg 10 in 40 ml Ether unter Argon und gibt 0,46 g (4,2 mmol) Trimethylchlorsilan, 375 mg (5,42 mmol) Imidazol und 0,6 ml Pyridin zu. Es wird über Nacht bei Raumtemperatur gerührt und anschließend mit Natriumchlorid-Lösung versetzt. Nach Extraktion mit Essigester, Waschen der organischen Phase mit Natriumchlorid-Lösung und Trocknen über Natriumsulfat entfernt man das Solvens und reinigt das Rohprodukt durch Chromatographie an Kieselgel mit Hexan/Essigester, wobei man 506 mg der Titelverbindung als farbloses Öl erhält.

$^1$H-NMR ($CDCl_3$): δ = 0,01 ppm (s, 9H, Si-$Me_3$); 0,61 (s,3H,H-18); 1,03 (d,J=7Hz,3H,H-21); 1,15 (s,6H,H-28 u. H-29)); 2,40 (dd,J=10,5, 7Hz,1H,H-14); 3,16 (m,2H,H-26); 3,88 (dd,J=12,5, 5,5Hz,1H,H-24); 3,92 (dd,J=12,5, 5,5Hz,1H, H-24');5,40 (dt,J=15,5, 5,5Hz,1H,H-23); 5,48 (dd,J=15,5, 9Hz,1H,H-22)

IR (KBr-Preßling): v=1710 $cm^{-1}$

**Beispiel 13**

[1R-[1α[R*-(E)],3aβ,7aα]]-1-[4-[2-Ethyl-2-[(trimethylsilyl)oxy]butoxy]-1-methyl-2-butenyl]-7a-methyloctahydro-4H-inden-4-on 14

Analog Beispiel 12 werden 425 mg (1,26 mmol) 11 mit 411 mg (3,67 mmol) Trimethylchlorsilan, 330 mg (4,77 mmol) Imidazol und 0,53 ml Pyridin umgesetzt, wobei man 474 mg der Titelverbindung als farbloses Öl erhält.

$^1$H-NMR ($CDCl_3$): δ = 0,00 ppm (s, 9H, Si-$Me_3$); 0,59 (s,3H,H-18); 0,74 (t,J=7Hz,6H,H-30 u. H-31); 1,00 (d,J=7Hz, 3H,H-21); 1,43 (q,J=7Hz,4H,H-28 u. H-29)); 2,40 (dd,J=10,5, 7Hz,1H,H-14); 3,14 (m,2H,H-26); 3,87 (dd,J=12,5, 5,5Hz, 1H,H-24); 3,92 (dd,J=12,5, 5,5Hz,1H,H-24');5,40 (dt,J=15,5, 5,5Hz,1H,H-23); 5,49 (dd,J=15,5, 9Hz,1H,H-22) IR (KBr-Preßling): v=1710 $cm^{-1}$

**Beispiel 14**

[1R-[1α[R*-(E)],3aβ,7aα]]-7a-Methyl-1-[1-methyl-4-[2-propyl-2-[(trimethylsilyl)oxy]pentoxy]-2-butenyl]octahydro-4H-inden-4-on 15

Analog Beispiel 12 werden 935 mg (2,56 mmol) 12 mit 803 mg (7,4 mmol) Trimethylchlorsilan, 671 mg (9,7 mmol) Imidazol und 1,01 ml Pyridin umgesetzt, wobei man 857 mg der Titelverbindung als farbloses Öl erhält.

$^1$H-NMR ($CDCl_3$): δ= 0,00 ppm (s, 9H, $SiMe_3$); 0,59 (s, 3H, H-18); 0,80 (t, J=7 Hz, 6H, H-32 u. H-33); 1,00 (d, J=7 Hz, 3H, H-21); 2,40 (dd, J=10,5, 7,5 Hz, 1H, H-14); 3,13 (m, 2H, H-26); 3,78 (dd, J=12,5, 5,5 Hz, 1H, H-24); 3,82 (dd, J=12,5, 5,5 Hz, 1H, H-24'); 5,39 (dt, J=15,5, 5,5 Hz, 1H, H-23); 5,48 (dd, J=15,5, 9 Hz, 1H, H-22)

**Beispiel 15**

(5Z,7E,22E)-(1S,3R)-24-(2-Hydroxy-2-methylpropoxy)-9,10-secochola-5,7,10(19),22-tetraen-1,3-diol 16

Man löst 83 mg (0,22 mmol) [3S-(1Z,3α,5β)]-[2-[3,5-Bis-[[dimethyl(1,1-dimethylethyl)-silyl]oxy]-2-methylencyclohexyliden]ethyl]diphenylphosphinoxid (M.R. Uskokovic et al. J. Org. Chem. 51, 3098 (1986)) in 2 ml Tetrahydrofuran und kühlt unter Argon auf -78°C. Nun werden 0,185 ml einer n-BuLi-Lösung (1,6 M Hexan) zugetropft. Nach 5 Minuten werden 85 mg (0,22 mmol) 13 in 2 ml Tetrahydrofuran zugetropft und 30 Minuten bei dieser Temperatur gerührt. Anschließend hydrolysiert man mit Kalium-Natriumtartrat/Kaliumhydrogencarbonat-Lösung, extrahiert mit Essigester, wäscht die organische Phase mit Natriumchlorid-Lösung und trocknet über Natriumsulfat. Das Lösungsmittel wird entfernt und der Rückstand unter Argon in 10 ml Tetrahydrofuran gelöst. Man gibt nun 0,5 ml (0,5 mmol) Tetrabutylammoniumfluorid-Lösung (1M in Tetrahydrofuran) zu und rührt 60 Minuten bei 60°C . Danach wird Natriumchlorid-Lösung zugegeben, mit Essigester extrahiert, die organische Phase mit Natriumchlorid-Lösung gewaschen, über Natriumsulfat getrocknet und das Solvens entfernt. Der Rückstand wird mehrmals an Kieselgel mit Hexan/Essigester gereinigt, wobei 10 mg der Titelverbindung als farbloser Schaum anfallen.

$^1$H-NMR ($CDCl_3$): δ = 0,59 ppm (s,3H,H-18); 1,06 (d,J=7Hz,H-21); 1,18 (s,6H,H-28 u. H-29); 3,21 (s,2H,H-26); 3,92 (dd,J=15, 5,5Hz,1H,H-24); 3,98 (dd,J=15, 5,5Hz,1H,H-24'); 4,18 (m,1H,H-3); 4,39 (m,1H,H-1); 4,96 (s,1H,H-19); 5,30 (s,1H,H-19'); 5,47 (dt,J=15,5, 5,5Hz,1H, H-23); 5,57 (dd,J=15,5, 7,5Hz,1H,H-22); 6,02 u. 6,38 (2x d,J=11Hz,je 1H,H-6 u. H-7).

**Beispiel 16**

(5Z,7E,22E)-(1S,3R)-24-(2-Ethyl-2-hydroxybutoxy)-9,10-secochola-5,7,10(19),22-tetraen-1,3-diol 17

Man setzt 100 mg (0,24 mmol) 14 analog Beispiel 15 mit 91 mg (0,24 mmol) [3S-(1Z,3α,5β)]-[2-[3,5-Bis-[[dimethyl (1,1-dimethylethyl)silyl]oxy]-2-methylencyclohexyliden]ethyl]diphenylphosphinoxid und 0,2 ml n-BuLi-Lösung sowie anschließend mit 0,54 ml (0,54 mmol) Tetrabutylammoniumfluorid-Lösung um und erhält nach mehrmaliger Chromatographie 19 mg der Titelverbindung als farblosen Schaum.

$^1$H-NMR ($CDCl_3$): δ = 0,53 ppm (s,3H,H-18); 0,80 (t,J=7Hz,6H,H-30 u. H-31); 1,02 (d,J=7Hz,3H-21); 1,38 (q,J=7Hz, 4H,H-28 u. H-29); 3,12 (m,2H,H-26); 3,80 (s,1H,tert.OH); 3,83 (dd,J=15, 5,5Hz,1H, H-24); 3,90 (dd,J=15, 5,5Hz,1H, H-24'); 4,00 (m,1H,H-3); 4,20 (m,1H,H-1); 4,42 (d,J=4Hz,1H,OH);4,72 (d,J=5Hz,1H,OH); 4,77 (s,1H,H-19); 5,21 (s, 1H,H-19'); 5,42 (dt,J=15,5, 5,5Hz,1H,H-23); 5,54 (dd,J=15,5, 7,5Hz,1H,H-22); 5,98 u. 6,18 (2x d,J=11Hz,je 1H,H-6 u. H-7).

**Beispiel 17**

(5Z,7E,22E)-(1S,3R)-24-(2-Hydroxy-2-propylpentoxy)-9,10-secochola-5,7,10(19),22-tetraen-1,3-diol 18

Man setzt 116 mg (0,24 mmol) 15 analog Beispiel 15 mit 300 mg (0,53 mmol) [3S-(1Z,3α,5β)]-[2-[3,5-Bis-[[dimethyl (1,1-dimethylethyl)silyl]oxy]-2-methylencyclohexyliden]ethyl]diphenylphosphinoxid und 0,2 ml n-BuLi-Lösung sowie anschließend mit 1,0 ml (1,0 mmol) Tetrabutylammoniumfluorid-Lösung um und erhält nach mehrmaliger Chromatogrphie 59 mg der Titelverbindung als farblosen Schaum.

$^1$H-NMR ($CD_2Cl_2$): δ= 0,58 (s, 3H, H-18); 0,90 (t, J=7 Hz, 6H, H-32 u. H-33); 1,05 (d, J=7 Hz, 3H, H-21); 3,20 (d, J=10 Hz, 1H, H-26); 3,25 (d, J=10 Hz, 1H, H-26'); 3,90 (dd, J=12,5, 5,5 Hz, 1H, H-24); 3,96 (dd, J=12,5, 5,5 Hz, 1H, H-24'); 4,18 (m, 1H, H-3); 4,39 (m, 1H, H-1); 4,97 (s, 1H, H-19); 5,30 (s, 1H, H-19'); 5,46 (dt, J=15,5, 6 Hz, 1H, H-23); 5,56 (dd, J=15,5, 7,5 Hz, 1H, H-22); 6,02 u. 6,38 (2x d, J=11 Hz, je 1H, H-6 u. H-7)

**Patentansprüche**

1. 22-En-25-oxa-Derivate in der Vitamin D-Reihe der allgemeinen Formel I

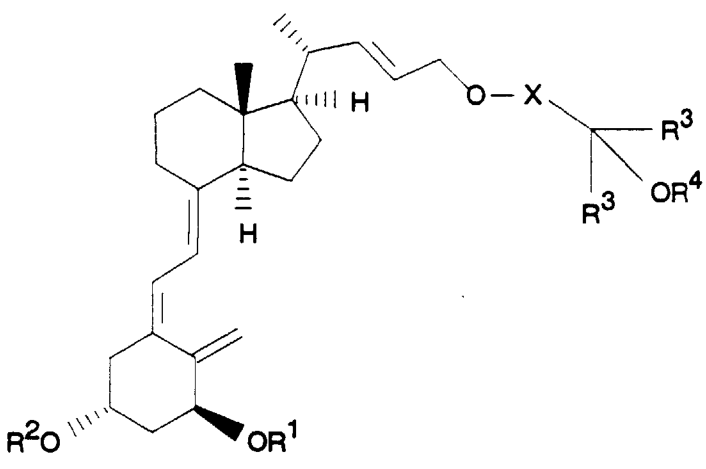

(I),

worin

| | |
|---|---|
| $R^1$, $R^2$ und $R^4$ | unabhängig voneinander je ein Wasserstoffatom, eine Alkanoylgruppe mit 1 bis 9 Kohlenstoffatomen oder eine Aroylgruppe, |
| $R^3$ | je ein Wasserstoffatom oder je eine lineare oder verzweigte Alkylgruppe mit 1 bis 4 Kohlenstoffatomen, je eine Trifluormethylgruppe oder einen gemeinsamen mit dem tertiären Kohlenstoffatom gebildeten gesättigten oder ungesättigten carbocyclischen oder heterocyclischen 3, 4, 5 oder 6-gliedrigen Ring und |
| X | einen Alkylenrest $-(CH_2)_n-$ mit n=1, 2 oder 3 |

bedeuten.

**2.** 22-En-25-oxa-Derivate nach Anspruch 1, dadurch gekennzeichnet, daß $R^1$, $R^2$ und $R^4$ Wasserstoffatome oder Alkanoylreste gesättigter Alkancarbonsäuren oder der Benzoylrest sind.

**3.** 22-En-25-oxa-Derivate nach Anspruch 1, dadurch gekennzeichnet, daß für beide $R^3$ Methyl-, Ethyl- oder Propylgruppen stehen.

**4.** 22-En-25-oxa-Derivate nach Anspruch 1, dadurch gekennzeichnet, daß beide $R^3$ gemeinsam mit dem tertiären Kohlenstoffatom für einen Cyclopropyl- oder Cyclopentylring stehen.

**5.** 22-En-25-oxa-Derivate nach Anspruch 1, dadurch gekennzeichnet, daß $R^1$, $R^2$ und $R^4$ je für ein Wasserstoffatom und $R^3$ je für eine Methyl-, Ethyl- oder Propylgruppe sowie X für eine Methylengruppe ($CH_2$) stehen.

**6.** Verbindungen gemäß Anspruch 1,

(5Z,7E,22E)-(1S,3R)-24-(2-Hydroxy-2-methylpropoxy)-9,10-secochola-5,7,10(19),22-tetraen-1,3-diol;
(5Z,7E,22E)-(1S,3R)-24-(2-Ethyl-2-hydroxybutoxy)-9,10-secochola-5,7,10(19),22-tetraen-1,3-diol;
(5Z,7E,22E)-(1S,3R)-24-(2-Hydroxy-2-propylpentoxy)-9,10-secochola-5,7,10(19),22-tetraen-1,3-diol.

**7.** Verfahren zur Herstellung der 22-En-25-oxa-Derivate der allgemeinen Formel I gemäß Anspruch 1, dadurch gekennzeichet, daß eine Verbindung der allgemeinen Formel XIV

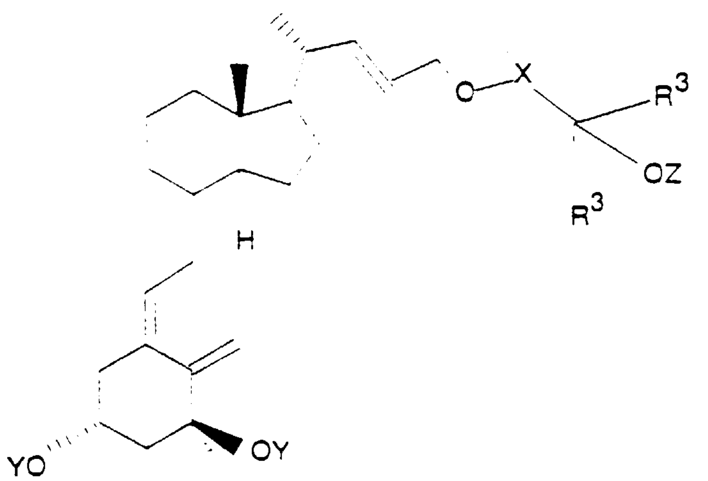

(XIV),

worin
Y Silyl-Hydroxyschutzgruppen und Z eine Silyl. Tetrahydropyranyl- oder Tetrahydrofuranylgruppe bedeuten, durch Abspaltung der Schutzgruppen in eine Verbindung der allgemeinen Formel I, worin $R^1$, $R^2$ und $R^4$ Wasserstoffatome bedeuten, und diese anschließend gewünschtenfalls durch Veresterung der freien Hydroxygruppen in eine Verbindung der allgemeinen Formel I, worin $R^1$, $R^2$ und $R^4$ $C_1$-$C_9$-Alkanoyl- oder Benzoylgruppen bedeuten, überführt wird.

**8.** Verfahren nach Anspruch 7, dadurch gekennzeichnet, daß die Schutzgruppenabspaltung mit Tetra-n-butyl-ammoniumfluorid/ HF oder HF-Pyridin-Komplex vorgenommen wird.

**9.** Pharmazeutische Präparate, enthaltend mindestens eine Verbindung der allgemeinen Formel I gemäß Anspruch 1 sowie einen pharmazeutisch verträglichen Träger.

**10.** Verwendung der Verbindungen der allgemeinen Formel I zur Herstellung von Arzneimitteln.

## Claims

1. 22-Ene-25-oxa derivatives in the vitamin D series of the general formula I

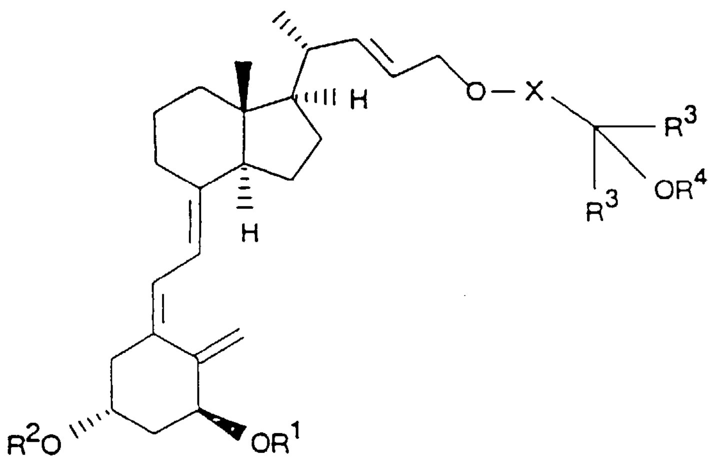

(I),

wherein

$R^1$, $R^2$ and $R^4$ are each independently of the others a hydrogen atom, an alkanoyl group having from 1 to 9 carbon atoms, or an aroyl group,
$R^3$ is each a hydrogen atom or $R^3$ is each a linear or branched alkyl group having from 1 to 4 carbon atoms, or $R^3$ is each a trifluoromethyl group or are a saturated or unsaturated carbocyclic or heterocyclic 3-, 4-, 5- or 6-membered ring formed together with the tertiary carbon atom, and
X is an alkylene radical $-(CH_2)_n$ in which n = 1, 2 or 3.

2. 22-Ene-25-oxa derivatives according to claim 1, characterised in that $R^1$, $R^2$ and $R^4$ are hydrogen atoms or alkanoyl radicals of saturated alkanecarboxylic acids or a benzoyl radical.

3. 22-Ene-25-oxa derivatives according to claim 1, characterised in that both radicals $R^3$ are selected from methyl, ethyl and propyl groups.

4. 22-Ene-25-oxa derivatives according to claim 1, characterised in that both radicals $R^3$, together with the tertiary carbon atom, represent a cyclopropyl or cyclopentyl ring.

5. 22-Ene-25-oxa derivatives according to claim 1, characterised in that $R^1$, $R^2$ and $R^4$ are each a hydrogen atom and each $R^3$ is a methyl, ethyl or propyl group and X is a methylene group ($CH_2$).

6. Compounds according to claim 1,

(5Z,7E,22E)-(1S,3R)-24-(2-hydroxy-2-methylpropoxy)-9,10-secochola-5,7,10(19),22-tetraene-1,3-diol;
(5Z,7E,22E)-(1S,3R)-24-(2-ethyl-2-hydroxybutoxy)-9,10-secochola-5,7,10(19),22-tetraene-1,3-diol;
(5Z,7E,22E)-(1S,3R)-24-(2-hydroxy-2-propylpentoxy)-9,10-secochola-5,7,10(19),22-tetraene-1,3-diol.

7. Process for the preparation of the 22-ene-25-oxa derivatives of the general formula I according to claim 1, characterised in that a compound of the general formula XIV

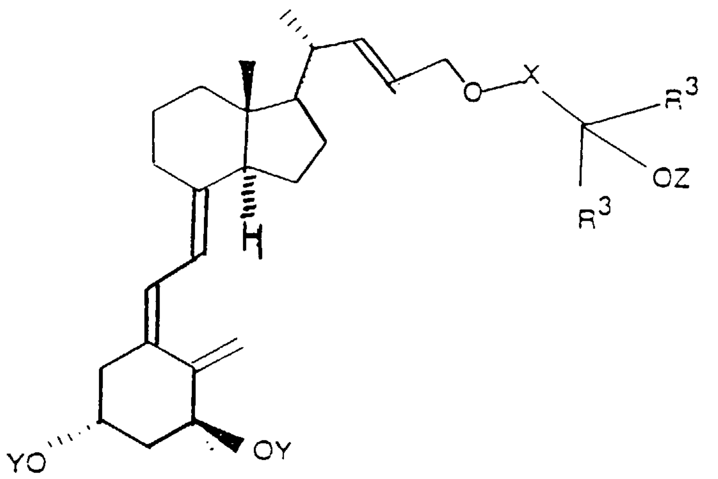

(XIV),

wherein
Y is silyl hydroxy-protecting groups and Z is a silyl, tertrahydropyranyl or tetrahydrofuranyl group, is converted by removing the protecting groups into a compound of the general formula I wherein $R^1$, $R^2$ and $R^4$ are hydrogen atoms, and then converting that compound, if desired, by esterifying the free hydroxy groups, into a compound of the general formula I wherein $R^1$, $R^2$ and $R^4$ are $C_1$-$C_9$alkanoyl or benzoyl groups.

**8.** Process according to claim 7, characterised in that the removal of the protecting groups is carried out using tetra-n-butyl-ammonium fluoride/HF or HF-pyridine complex.

**9.** Pharmaceutical preparations comprising at least one compound of the general formula I according to claim 1 and a pharmaceutically acceptable carrier.

**10.** Use of the compounds of the general formula I for the preparation of medicaments.

## Revendications

**1.** Dérivés 22-èn-25-oxa de la série de la vitamine D, de formule générale I

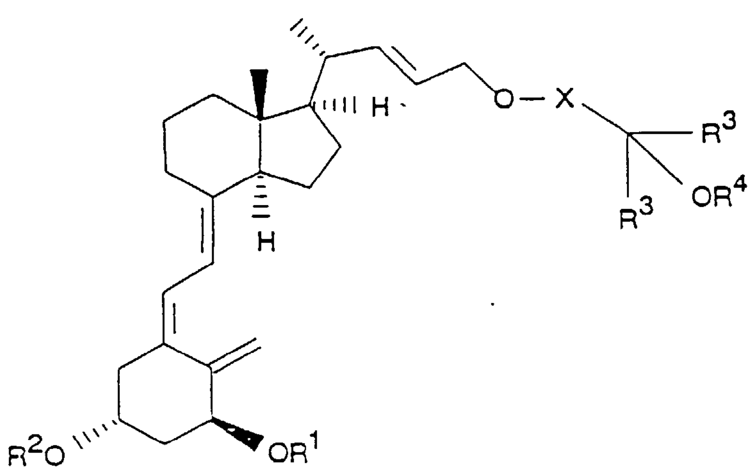

(I),

dans laquelle

- $R^1$, $R^2$ et $R^4$, indépendamment l'un de l'autre, représentent chacun un atome d'hydrogène, un groupe alcanoyle avec 1 à 9 atomes de carbone ou un groupe aroyle,

- les $R^3$ représentent chacun un atome d'hydrogène ou chacun un groupe alkyle, linéaire ou ramifié, avec 1 à 4 atomes de carbone, chacun un groupe trifluorométhyle ou un cycle à 3, 4, 5 ou 6 chaînons, carbocyclique ou hétérocyclique, saturé ou insaturé, formé ensemble avec l'atome de carbone tertiaire et
- X représente un radical alkylène $-(CH_2)n-$ avec n = 1, 2 ou 3.

**2.** Dérivés 22-èn-25-oxa selon la revendication 1, caractérisés en ce que $R^1$, $R^2$ et $R^4$ sont des atomes d'hydrogène ou des radicaux alcanoyle d'acides alcanecarboxyliques saturés ou le radical benzoyle.

**3.** Dérivés 22-èn-25-oxa selon la revendication 1, caractérisés en ce que les deux $R^3$ représentent des groupes méthyle, éthyle ou propyle.

**4.** Dérivés 22-èn-25-oxa selon la revendication 1, caractérisés en ce que les deux $R^3$ représentent ensemble avec l'atome de carbone tertiaire un cycle cyclopropyle ou cyclopentyle.

**5.** Dérivés 22-èn-25-oxa selon la revendication 1, caractérisés en ce que $R^1$, $R^2$ et $R^4$ représentent chacun un atome d'hydrogène et les $R^3$ représentent chacun un groupe méthyle, éthyle ou propyle, ainsi que X représente un groupe méthylène ($CH_2$).

**6.** Composés selon la revendication 1 :

- (5Z,7E,22E)-(1S,3R)-24-(2-hydroxy-2-méthylpropoxy)-9,10-secochola-5,7,10(19),22-tétraèn-1,3-diol;
- (5Z,7E,22E)-(1S,3R)-24-(2-éthyl-2-hydroxybutoxy)-9,10-secochola-5,7,10(19),22-tétraèn-1,3-diol ;
- (5Z,7E,22E)-(1S,3R)-24-(2-hydroxy-2-propylpentoxy)-9,10-secochola-5,7,10(19),22-tétraèn-1,3-diol.

**7.** Procédé de préparation des dérivés 22-èn-25-oxa de formule générale I selon la revendication 1, caractérisé en ce qu'on transforme un composé de formule générale XIV

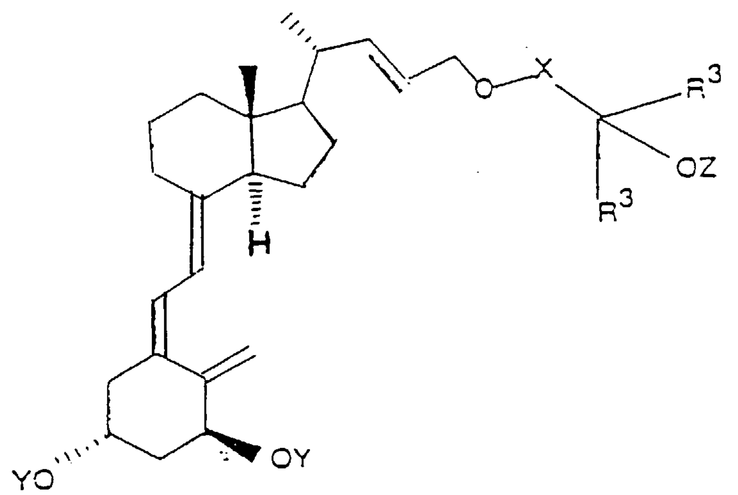

(XIV).

dans laquelle
Y représente des groupes silyle protecteurs des hydroxy et Z représente un groupe silyle, tétrahydropyranyle ou tétrahydrofuranyle,
par clivage des groupes protecteurs, en un composé de formule générale I dans lequel $R^1$, $R^2$ et $R^4$ représentent des atomes d'hydrogène, et, si on le souhaite, on transforme celui-ci ensuite, par estérification des groupes hydroxy libres, en un composé de formule générale I, dans lequel $R^1$, $R^2$ et $R^4$ représentent des groupes alcanoyle en $C_1$-$C_9$ ou benzoyle.

**8.** Procédé selon la revendication 7, caractérisé en ce que le clivage des groupes protecteurs est effectué au moyen de fluorure de tétra-n-butylamnonium/HF ou de complexe HF-pyridine.

**9.** Préparations pharmaceutiques contenant au moins un composés de formule générale I selon la revendication 1, ainsi qu'un excipient pharmaceutiquement acceptable.

**10.** Utilisation des composés de formule générale I pour préparer des médicaments.